Europäisches Patentamt

(19) European Patent Office

Office européen des brevets

(11) Publication number : **0 418 739 B1**

# (12) EUROPEAN PATENT SPECIFICATION

(45) Date of publication of patent specification :
01.02.95 Bulletin 95/05

(51) Int. Cl.⁶ : **G01N 33/53**, // G01N33/569

(21) Application number : **90117670.1**

(22) Date of filing : **13.09.90**

(54) **Oral immunoglobulin collection for immunoassay.**

(30) Priority : **21.09.89 US 410401**
**28.02.90 US 486415**

(43) Date of publication of application :
**27.03.91 Bulletin 91/13**

(45) Publication of the grant of the patent :
**01.02.95 Bulletin 95/05**

(84) Designated Contracting States :
**AT BE CH DE DK ES FR GB IT LI LU NL SE**

(56) References cited :
**WO-A-88/04431**
**WO-A-88/07680**

(73) Proprietor : **EPITOPE, INC.**
**15425-E S.W. Koll Parkway**
**Beaverton Oregon 97006 (US)**

(72) Inventor : **Goldstein, Andrew S.**
**6944 S.W. Windemere Loop,**
**Portland, Oregon 97351 (US)**
Inventor : **Gavojdea, Stefan**
**12154 S.W. Millview Court**
**Tigard, Oregon 97223 (US)**

(74) Representative : **von Füner, Alexander, Dr. et al**
**Patentanwälte v. Füner, Ebbinghaus, Finck**
**Mariahilfplatz 2 & 3**
**D-81541 München (DE)**

## Description

The present invention relates to the field of immunological testing. In particular, a system for analyzing immunoglobulins extracted from the oral cavity is disclosed.

The immune system of the mouth not only interacts with the general immune system of the body, but also has its own centralized center for antigen-antibody response. Within the oral cavity is found extraoral lymph nodes and intraoral lymphoid aggregations. The extraoral lymph nodes are involved in the drainage of the oral mucosa, gum and teeth. However, the function of the intraoral lymphoid tissue is little understood.

The extraoral lymph nodes include a fine network of lymph capillaries which are superficially located in the mouth, palate, cheeks, lips, gingiva, and pulp of the teeth. The capillaries join to larger lymph vessels which originate from a network deep in the muscle of the tongue and other structures. An antigen can gain entry into the oral lymphatic system directly through the capillaries or be transported there by phagocytes. Once inside the network, the antigen can induce an immune response.

Included in the intraoral lymphoid tissue are generally four distinct tissue aggregations: (a) the tonsils, (b) scattered submucosal lymphoid aggregations, (c) salivary gland lymphoid tissue, and (d) gingival lymphoid tissue.

The tonsils (palatine and lingual) primarily produce B-cells and T-cells which are generally contained within a cap of lymphocytes and plasma cells. Antigen typically gains entry into the tonsils through a distinct epithelial region wherein the antigen can come into contact with the T- and B-cells to stimulate an immune response. The predominant type of antibody formed in the tonsils is found to be IgG followed, in order, by IgA, IgM, IgD and IgE.

Scattered submucosal lymphoid cells have not been extensively studied. These cell masses are histologically similar to tonsillar tissue.

Both the major salivary glands (parotid, submandibular and sublingual) and the minor salivary glands have been found to contain lymphocytes and plasma cells. Most of the plasma cells secrete IgA and some IgG or IgM. The IgA synthesized in the salivary glands has a dimeric structure. This type of IgA is referred to as secretory IgA (sIgA) and is the major immunoglobulin component in saliva.

Both T-cells and B-cells are found in the gingival lymphoid tissue. In subjects having clinically normal gingival tissue, T-cells predominate. During an infectionary period, such as during the development of gingivitis, B-cells have been found to predominate.

Plasma cells are also found in the gingival lymphoid tissue. Clusters of these cells are generally located near the blood vessels and predominately produce IgG. To a lesser extent, IgA and IgM are also manufactured. More importantly, Brandtzaeg et al. in, Human Saliva: Clinical Chemistry and Microbiology edited by Jorma 0. Tenovuo, have shown that the immunoglobulins from the secretions from the gingival tissue area are directly related to the immunoglobulins found in the blood.

Because of the association between immunoglobulins of the blood and saliva, as well as the occurrence of sIgA peculiar to salival fluid, antigen-antibody tests have been conducted on the saliva to assess the value of such tests as a screening tool for diseases.

Collection of saliva from the salivary glands is complicated by the low volumes secreted, the diverse anatomic dispersion of the glands, and the relatively high viscosity of the fluid. Most techniques for collection involve the use of capillary tubes, suction into micropipettes, chewing on paraffin or aspiration into polypropylene syringes. These methods, however, are limited in that viscosity of the saliva makes the recovery of bubble-free material by these techniques difficult. Other methods of collection have been suggested to eliminate or at least reduce the quantity of bubbles in the sample. Among such methods include collecting saliva in the mouth by direct absorption with a sponge or flexible wad of osmotic membrane. After absorption, the saliva can be separated from the absorptive material by centrifugation or by compressing the absorptive material. However, absorption is generally accomplished by using cotton, nylon, or polyester as the absorptive material. These materials can non-specifically bind proteins which can result in an undesirably low recovery of immunoglobulins.

Testing of salivary specimens has not been extensively developed. In addition to problems with collection, the samples collected by the known methods typically contain 0.01-0.1% of the immunoglobulin found in blood serum. Because of the reduced immunoglobulin content of saliva, it has been necessary to use more accurate antigen-antibody assay methods in screening patients for disease. Parry et al., "Rational Programme for Screening Travellers for Antibodies to Hepatitis A Virus", The Lancet, June 25, 1988, have discussed such methods and have found that the more accurate IgG-capture radioimmunoassay (GACRIA) test is preferable to avoid false indications which may occur in less accurate methods. Of course, more accurate testing procedures usually require added time and expense to achieve the test results.

Fig. 1a is a side view of the pad and pad holder.

Fig. 1b is a top view of the pad and pad holder.

Fig. 2 is a top view of the pad removal device.

Fig. 3 details a preferred embodiment of a container for storing the pad.

Fig. 4 is a flow diagram demonstrating how the pad is to be placed and stored in the container.

In order to eliminate or greatly reduce the problems inherent in antigen-antibody analysis of salival fluid, the present invention provides a method for collecting immunoglobulins from the oral cavity in a manner highly desirable for use in immunoassays. This method can be accomplished with the aid of a hypertonic solution. In a first aspect the method concerns rinsing the oral cavity with an aqueous solution, preferably a hypertonic solution, collecting the solution after rinsing and analyzing immunoglobulin content of the collected solution. In a second aspect, the method concerns placing an oral immunoglobulin collecting pad, which has been treated with a hypertonic solution, in the oral cavity to absorb a sufficient quantity of oral immunoglobulin for immunological testing. The use of the rinse or pad results in a yield of immunoglobulins greater than would be expected and can incorporate basic antigen-antibody testing techniques as a screening tool for diseases.

The hypertonic solution of the present invention can also include additives to further provide for an optimal yield in salivary immunoglobulin content. Such additives can include compounds which maintain the correct pH, compounds which preserve the oral immunoglobulins, or compounds which inhibit the growth of organisms. The combination of such compounds provides for the collection of a salival fluid specimen which requires minimum manipulation in preparing the specimen for testing.

The present invention is concerned with collecting oral immunoglobulins for immunological testing. A rinse or a treated pad is used to collect a specimen having a high concentration of immunoglobulins. High levels of immunoglobulins from the oral cavity are considered to be concentrations in excess of 50 $\mu$g total Ig per ml. The specimen can be subjected to a basic immunological testing technique which can be used as a tool for screening a patient for diseases.

The solution to be used as the rinse or in the pad of the present invention is a hypertonic solution. Although a non-hypertonic solution such as water may be used, it has been found that immunoglobulin production from salivation rapidly declines in concentration using such a solution. However, the use of a hypertonic solution results in a constant production of immunoglobulin from other sources within the oral cavity, those sources not being completely understood. By using a hypertonic solution, it is possible to gain an increase of as much as 8-16 times more immunoglobulin than by using distilled water.

A hypertonic solution is a salt solution which has an ionic strength exceeding that found in blood. In general, salts used in the preparation the hypertonic solution of the present invention are present in an amount of from 1.5% to 5% by weight, preferably 3.5% by weight.

Salts which can be used in the preparation of the hypertonic solution include alkali metal compounds as well as alkaline earth metal compounds. Preferred salts include sodium chloride, potassium chloride, magnesium sulfate, magnesium chloride and calcium chloride. Sodium chloride is found to be the least toxic, least expensive and most palatable.

The hypertonic solution of the present invention can also include a compound or ingredient for stimulating salivation. The compounds capable of stimulating salivation are found to exhibit a sour taste. These compounds include weak organic acids. Preferred among the weak organic acids are citric acid, ascorbic acid and acetic acid. It is preferred to use citric acid and ascorbic acid at a concentration of between 0.05% and 0.5% by weight. The preferable range for acetic acid is between 0.5% and 3.0% by weight.

In order to minimize immunoglobulin degradation in a collected specimen, the hypertonic solution of the present invention can include a preservative. Such a preservative can act to inhibit proteolytic enzymatic activity which can be responsible for the destruction of antibody molecules. Compounds contemplated as a preservative include anti-bacterial agents, anti-fungal agents, bacteriostatic agents, fungistatic agents, and enzyme inhibitors. In a preferred embodiment benzoic acid, sorbic acid or the salts thereof are used as anti-fungal agents. As bacteriostatic agents, salts in high concentration and compounds capable of maintaining the hypertonic solution at low pH are contemplated. Such salts include thimerosal (or merthiolate), phenyl mercuric acetate, phenyl mercuric nitrate and sodium azide. Other preferred preservatives include preservatives which are typically used in medicines and mouthwashes. Examples include ethyl alcohol and chlorhexidine gluconate. Another class of preferred anti-microbial agents are detergents which can be used as topical germicides or in mouthwashes. An example is benzalkonium chloride. It is preferred to use these preservatives in a range of 0.01% to 0.2% by weight.

There are two approaches to collecting a specimen according to the present invention. In a first aspect, a hypertonic solution is placed in the oral cavity and vigorously rinsed. The preferred volume of rinse is 4-5 ml. Larger volumes further dilute the antibody, smaller volumes make it difficult to rinse vigorously. Generally, the longer the solution is rinsed, the greater the content of immunoglobulin in the specimen. Because of practical limitations, such as fatigue, it is difficult to rinse for more than two minutes. Rinsing time should be at least one minute and preferably two minutes. After rinsing is complete, the specimen is collected and made ready for testing.

The preferred oral rinse solution has the following composition:

| component | conc. (wt.%) |
| --- | --- |
| sodium chloride | 3.0% |
| citric acid | 0.2% |
| sodium benzoate | 0.05-0.1% |
| potassium sorbate | 0.05-0.1% |

distilled water
addition of 0.1N sodium
hydroxide to increase pH
to about 6.5

The specimen can either be tested without further treatment or passed through a low protein binding filter. This filter will aid in removing particulates. A 5.0 micron filter is preferable. If the specimen is clear, a variety of immunological assays can be performed without further treatment. If the specimen is not clear, additional steps such as centrifugation (minimum of 400 x g for 10 minutes) or filtering can be performed. Preferable immunological assays include enzyme-linked immunoabsorbent assay (ELISA) and Western Blot assay. These methods are particularly useful in determining the presence of antibody specific to Human Immunodeficiency Virus (HIV), Hepatitis A, Cytomegalovirus, Rubella, Herpes and Calicivirus.

In a second aspect of the present invention, a pad containing the salts of the hypertonic solution is used to absorb saliva and mucosal secretions from the oral cavity. The pad is made of an absorbent material which can be effectively placed into the oral cavity. A plastic or carbohydrate material such as cellulose can be used as the absorbent material, but a thick, absorbent cotton paper is preferred. An example of a thick, absorbent cotton paper is product #300 manufactured by Schleicher and Schuell in Keene, New Hampshire.

The hypertonic solution of the present invention is applied to the pad by dipping the pad into the hypertonic solution so that the salts of the solution can be absorbed into and onto the pad, removing the pad from the solution and allowing the pad to dry. Typically, the pad is dipped into the hypertonic solution and 1 ml of solution is absorbed. Excess liquid is shaken off and the pad is placed into a forced air, convection drying oven at 50°C for 2 hours. After drying, there will be formed a specially treated pad which comprises the salts of the hypertonic solution of the present invention. It is preferred that, as preservatives, such salts as benzalkonium chloride, acetyl pyridinium chloride or chlorhexidine gluconate be used in the preparation of the pad.

Most materials from which the pad is made can non-specifically bind protein. Thus, some immunoglobulins can undesirably bind to the pad and it is desired to block proteins from binding to the pad by using a blocking agent. Non-specific binding is not normally a problem in the collection of blood samples since blood contains its own blocking agent (i.e., human serum albumin).

To reduce non-specific binding in the collection of oral specimens, a blocking agent can be added to the hypertonic solution to be incorporated into the pad. A blocking agent is generally a soluble protein which is used to prevent non-specific binding of another protein to a solid surface. Compounds which can be added as blocking agents include albumin and gelatin, but any water soluble, non-toxic protein can be used as a blocking agent as long as the protein does not adversely affect antibody molecules. It is preferred to use bovine gelatin. In general, blocking agents can be added to the hypertonic solution of the present invention at a concentration of between 0.01% and 0.2% by weight. The contents of the hypertonic solution are then incorporated into the pad as described above.

The preferred solution to be used in the preparation of the pad has the following composition:

| component | conc. (wt.%) |
| --- | --- |
| sodium chloride | 3.0% |
| sodium benzoate | 0.1% |
| potassium sorbate | 0.1% |
| bovine gelatin | 0.1% |

distilled water
addition of 0.1N sodium
hydroxide to increase pH
to 6.5

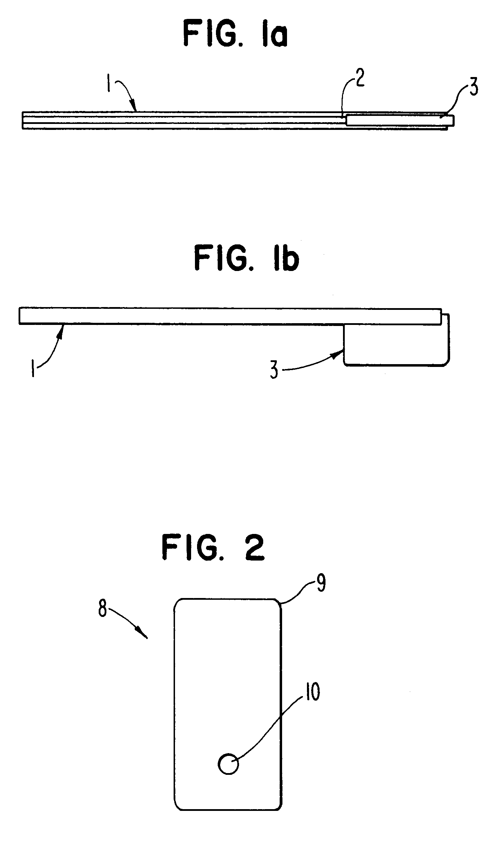

To collect immunoglobulin from the oral cavity, the pad can be placed into the mouth with the aid of a holder. The pad and holder are shown in Figs. 1a and 1b. The pad holder **1** can be a hollow, plastic stick having a groove **2** at one end. The pad **3** is inserted into the groove and the holder can be manipulated to place the pad into the oral cavity, preferably between the lower gums and cheek. Placement of the pad between the lower cheek and gums facilitates absorption of secretions originating from gingival lymphoid tissue as well as secretions from submucosal lymphoid tissue and salivary gland lymphoid tissue. It is preferable that the specimen be collected by rubbing the pad back and forth between the gums and cheek for about ten seconds and then holding the pad in position for about two minutes.

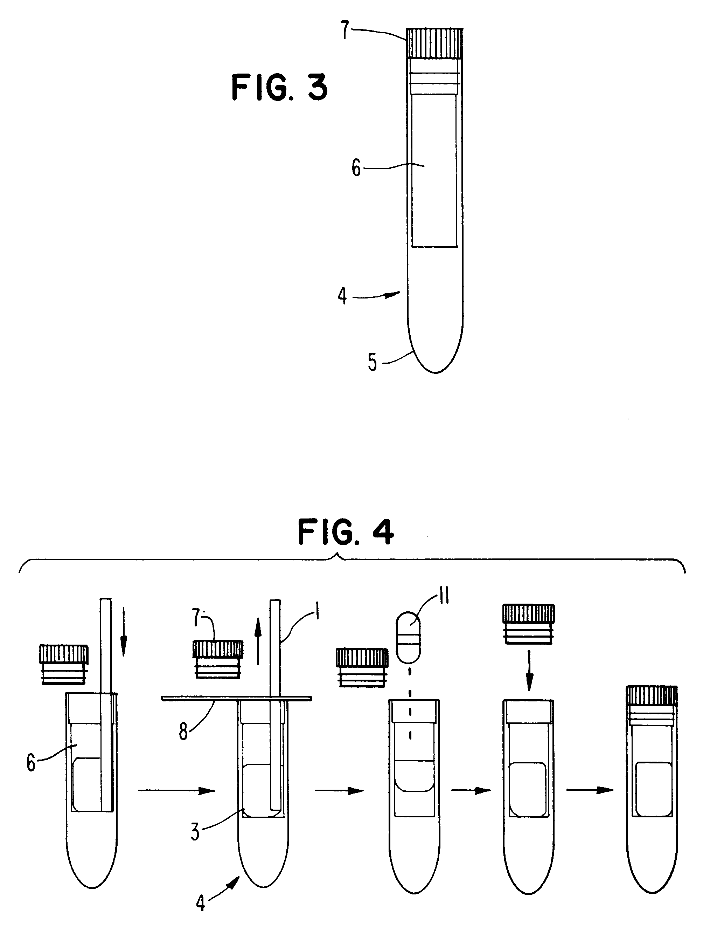

After the specimen has been collected, the pad is stored in a container until immunological testing can be performed. A preferred container is shown in Fig 3. It is desired that the container **4** have a centrifuge tube **5** as an outer portion of the container, and that an inner portion of the container have an inner tube **6** which mounts into the centrifuge tube. The pad is to be placed into the inner tube, and the contents therein are secured by a tube cap **7**.

To place the pad in the inner tube, a pad removal device is used. The device is shown in Figs. 2 and 4. The pad removal device **8** is preferably a disk **9** which has an opening **10** through which the pad holder **1** can be inserted.

The pad can be inserted into the the inner tube and prepared for storage in advance of immunological testing in the manner illustrated in Fig. 4. The tube cap **7** is removed from the container **4**, and the pad **3** and holder are inserted into the inner tube **6**. The pad removal device **8** is placed over the holder. Then, the pad holder is inserted through the opening of the pad removal device and the holder pulled through the opening to remove the pad. Once the pad is placed in the inner tube, a preservative solution **11** is added. Such a preservative solution can act to inhibit enzymatic activity which can be responsible for the destruction of antibody molecules or can function as an anti-microbial agent.

Compounds contemplated for use in the inner tube as a preservative include anti-bacterial agents, antifungal agents, bacteriostatic agents, fungistatic agents, and enzyme inhibitors. As an antibacterial agent, it is preferred to use chlorhexidine gluconate or thimerosal.

The preservative solution to be used in the inner tube can contain one or a combination of the preservatives which can be incorporated into the hypertonic solution of the present invention. In general, the preservatives are included in a concentration which limits microbial contamination and does not adversely effect the immunoglobulins absorbed into the pad.

The preservative solution to be used in the inner tube can also contain a detergent which improves removal of antibody from the pad during centrifugation. Tween® 20 (polyoxyethylene sorbitan monooleate) is a preferred detergent since it can also prevent non-specific binding of antibody to a solid surface. It is preferred to use a combination comprising 0.01%-0.2% chlorhexidine gluconate and 0.2%-0.7% Tween® 20. A combination comprising 0.1% chlorhexidine gluconate and 0.5% Tween® 20 is most preferred.

After the preservative solution is added to the inner tube, the tube cap is inserted into the container to seal in the contents. The pad can be stored in this manner for several days until immunological testing can be initiated.

To simplify the collection and analysis of an oral specimen using the pad collection system, a kit can be provided. The kit can include a combination of the treated pad and implements used to collect and prepare the oral specimen for further immunological analysis. A preferred embodiment of the kit includes the treated pad **3** and pad holder **1**; the container **4** having the inner tube **6**, the outer tube **5** and the cap **7**; the pad removal device **8**; and the storage preservative **11**.

The following examples show the effectiveness of the hypertonic solution of the present invention and the pad incorporating the solution of the present invention.

EXAMPLE 1

Oral Immunoglobulin Titers as a Function of Rinsing Time

The preferred oral rinse solution of the present invention is prepared (the pH being adjusted to about 6.0). Samples are analyzed for total immunoglobulin content among thirteen normal individuals (i.e. seronegative for HIV antibody). Five of the individuals rinse for 10 seconds, four rinse for 30 seconds and four rinse for 60 seconds. A dot blot immunoassay is used to measure immunoglobulin. Each rinse sample is serially diluted and 1 microliter dots are placed on a nitrocellulose test strip. The last dilution to produce a visible dot after performing the immunoassay, indicates the approximate concentration of antibody in the undiluted rinse.

The results are as follows:

| SPECIMEN # | RINSE TIME (SECONDS) | ANTIBODY TITER IN RINSE (MICROGRAMS PER MILLITER) |
|---|---|---|
| 1 | 10 | 42 |
| 2 | 10 | 42 |
| 3 | 10 | 21 |
| 4 | 10 | 21 |
| 5 | 10 | 21 |
| 6 | 30 | 84 |
| 7 | 30 | 42 |
| 8 | 30 | 42 |
| 9 | 30 | 42 |
| 10 | 60 | 84 |
| 11 | 60 | 84 |
| 12 | 60 | 84 |
| 13 | 60 | 84 |

This study demonstrates that increasing rinse time results in greater concentrations of total immunoglobulin.

EXAMPLE 2

Western Blot Test for HIV Antibody - Comparing Serum, Whole Saliva and Rinse

The preferred oral rinse solution of the present invention (adjusted to a pH of 6.0) is used to test a cohort of 15 individuals (7 seropositive, a seronegative). The results of the oral rinse solution of the present invention are compared for specific antibody levels in serum, whole saliva and rinse, using the Western Blot technique. This method shows the relative strength of antibody reaction against the major components of the AIDS virus (i.e., core, envelope, polymerase). The following results are obtained:

| PATIENT | SPECIMEN | SEROSTATUS | RELATIVE REACTION STRENGTH* |
|---------|----------|------------|------------------------------|
| 166 | blood | (-) | (-) |
|  | saliva |  | (-) |
|  | rinse |  | (-) |
| 167 | blood | (-) | (-) |
|  | saliva |  | (-) |
|  | rinse |  | (-) |
| 168 | blood | (-) | (-) |
|  | saliva |  | (-) |
|  | rinse |  | (-) |
| 169 | blood | (+) | 3+ |
|  | saliva |  | 2+ |
|  | rinse |  | 4+ |
| 170 | blood | (+) | 4+ |
|  | saliva |  | +/- |
|  | rinse |  | 4+ |
| 171 | blood | (-) | (-) |
|  | saliva |  | (-) |

**(Example 2 continued)**

| PATIENT | SPECIMEN | SEROSTATUS | RELATIVE REACTION STRENGTH* |
|---------|----------|------------|------------------------------|
|         | rinse    |            | (-) |
| 172     | blood    | (+)        | 3+ |
|         | saliva   |            | 2+ |
|         | rinse    |            | 4+ |
| 173     | blood    | (+)        | 2+ |
|         | saliva   |            | +/- |
|         | rinse    |            | 2+ |
| 174     | blood    | (+)        | 4+ |
|         | saliva   |            | 3+ |
|         | rinse    |            | 3+ |
| 175     | blood    | (+)        | 3+ |
|         | saliva   |            | 4+ |
|         | rinse    |            | 4+ |
| 176     | blood    | (-)        | (-) |
|         | saliva   |            | (-) |
|         | rinse    |            | (-) |
| 177     | blood    | (-)        | (-) |
|         | saliva   |            | (-) |
|         | rinse    |            | (-) |
| 178     | blood    | (-)        | (-) |
|         | saliva   |            | (-) |
|         | rinse    |            | (-) |
| 179     | blood    | (+)        | 1+ |
|         | saliva   |            | 1+ |
|         | rinse    |            | 1+ |
| 180     | blood    | (-)        | (-) |
|         | saliva   |            | (-) |
|         | rinse    |            | (-) |

*Relative reaction strength is defined as follows:

(-) = no bands visible by Western Blot;

+/- = 2 or fewer very weak bands visible;

1+ = 2 or more bands visible, at least 1 clearly;

2+ = 3 or more bands visible, at least 2 clearly;

3+ = all major bands visible, 1 or more weak;

4+ = all major bands strongly visible.

Data from Example 2 show that the rinse method of saliva collection is as effective as blood and more effective than whole saliva in detecting HIV antibodies in patient with AIDS. Patients without AIDS do not show any false positive results.

EXAMPLE 3

Anti-HIV Oral Antibody Stability - Water Versus Oral Rinse

The stability of oral-derived antibodies from an AIDS patient is tested by incubating samples derived by a distilled water rinse versus the preferred rinse solution (adjusted to a pH of 6.0). Western Blots are performed on both rinses after a storage of antibody at 37°C. The following results demonstrate that the preferred rinse provides a more stable specimen than a water rinse.

| LENGTH OF STORAGE (DAYS AT 37°C) | RELATIVE REACTION STRENGTH[*] | |
|---|---|---|
| | DISTILLED WATER | RINSE |
| 4 | 2+ | 5+ |
| 14 | +/- | 5+ |
| 26 | - | 4+ |

[*]Relative reaction strength is defined as follows:

(-) = no bands visible by Western Blot;

+/- = 2 or fewer very weak bands visible;

2+ = 2 or more bands visible, at least 1 clearly;

3+ = 3 or more bands visible, at least 2 clearly;

4+ = all major bands visible, 1 or more weak;

5+ = all major bands strongly visible.

EXAMPLE 4

ELISA Test Data for HIV Antibody - Comparing Serum, Oral Rinse and Oral Pad Eluate

The preferred oral rinse solution of the present invention is prepared except and the pH is adjusted to 6.0. A pad is prepared using the preferred pad preparation solution of the present invention. Fifteen individuals (12 seropositive, 3 seronegative) are compared for specific antibody levels in serum, rinse derived oral immunoglobulin and pad derived oral immunoglobulin. A commercial ELISA test is used to detect HIV antibody. This test method shows the relative titer of antibody against the AIDS virus. The results show that in most cases, the pad yields higher antibody concentrations than the rinse.

| PATIENT | SPECIMEN | SEROSTATUS | ELISA RESULTS (O.D. VALUE) (HIGHER NUMBER = STRONGER) |
|---------|----------|------------|------------------------------------------------------|
| 236 | blood* | (+) | >2.0 |
|  | rinse** |  | >2.0 |
|  | pad*** |  | >2.0 |
| 237 | blood | (+) | >2.0 |
|  | rinse |  | 1.735 |
|  | pad |  | >2.0 |
| 238 | blood | (−) | 0.077 |
|  | rinse |  | 0.050 |
|  | pad |  | 0.051 |
| 239 | blood | (+) | >2.0 |
|  | rinse |  | 1.423 |
|  | pad |  | >2.0 |
| 240 | blood | (−) | 0.088 |
|  | rinse |  | 0.051 |
|  | pad |  | 0.060 |
| 241[†] | blood | (−) | 0.751 |
|  | rinse |  | 0.061 |
|  | pad |  | 0.052 |
| 242 | blood | (+) | >2.0 |
|  | rinse |  | 1.537 |
|  | pad |  | >2.0 |
| 243 | blood | (−) | 0.062 |
|  | rinse |  | 0.045 |
|  | pad |  | 0.046 |
| 244 | blood | (+) | >2.0 |
|  | rinse |  | >2.0 |
|  | pad |  | 1.981 |

2(Example 4 continued)

| PATIENT | SPECIMEN | SEROSTATUS | ELISA RESULTS (O.D. VALUE) (HIGHER NUMBER = STRONGER) |
|---------|----------|------------|---------------------------------------|
| 245 | blood | (+) | >2.0 |
|  | rinse |  | 1.742 |
|  | pad |  | >2.0 |
| 246 | blood | (+) | >2.0 |
|  | rinse |  | 1.431 |
|  | pad |  | >2.0 |
| 247 | blood | (+) | >2.0 |
|  | rinse |  | 1.368 |
|  | pad |  | >2.0 |
| 248 | blood | (+) | >2.0 |
|  | rinse |  | 1.492 |
|  | pad |  | 1.825 |
| 249 | blood | (+) | >2.0 |
|  | rinse |  | 0.294 |
|  | pad |  | 0.740 |
| 250 | blood | (+) | >2.0 |
|  | rinse |  | >2.0 |
|  | pad |  | >2.0 |
| 251 | blood | (+) | >2.0 |
|  | rinse |  | >2.0 |
|  | pad |  | >2.0 |

\* Positive blood test results are numbers greater than 0.226.

\*\* Positive rinse test results are numbers greater than 0.241.

\*\*\* Positive pad test results are numbers greater than 0.351.

† This specimen shows a false positive reaction in the serum and a true negative reaction in the rinse and pad.

A negative Western Blot plus additional ELISA testing confirms the false positive reaction in the serum.

EXAMPLE 5

--Oral Immunoglobulin Stability Comparison--

Pad Stored at 37°C With and Without Preservative A pad is prepared using the preferred pad preparation solution of the present invention. An HIV positive individual is tested to compare immunoglobulin stability of the pad when stored in a distilled water solution and when stored in a preservative solution. The individual is tested by placing two pads in the mouth, one on each side, between the lower cheek and gum. One pad is treated with a gelatin blocking agent. The other pad is treated with the preferred pad preparation solution of the present invention. After removing the pads from the mouth of the individual, the material collected in the gelatin treated pad is eluted with a 0.5 ml solution of 0.3% Tween® 20. The material collected in the pad treated with the preferred pad preparation solution is eluted with a 0.5 ml solution of 0.2% chlorhexidine gluconate and 0.3% Tween® 20. The extracts from each pad is divided into five aliquots. One of the aliquots from each pad is frozen immediately and labelled as time "0" specimen. The other aliquots are stored at 37°C and tested by ELISA at periods of 1, 3, 7 and 14 days. The time "0" specimen is then thawed and tested by ELISA. The results, as indicated below, show improved preservation of oral immunoglobulin when the preservative solution is used.

| Number of days stored at 37°C | Preserved specimen ELISA O.D. | Unpreserved specimen ELISA O.D. |
|---|---|---|
| 0 | 1.91 | 1.70 |
| 1 | 1.87 | 1.61 |
| 3 | 1.71 | 1.17 |
| 7 | 1.70 | 1.02 |
| 14 | 1.52 | 0.67 |

Although many embodiments of the present invention are disclosed, it is to be understood that these embodiments are not limiting. For example, many components can be incorporated into the hypertonic solution of the present invention. The disclosed components represent specific examples which are capable of yielding an increased immunoglobulin concentration in oral specimens. Of course, any list of components cannot be exhaustive and alternatives can be predicted within the scope of the contemplated invention.

## Claims

1. A method of collecting immunoglobulins for immunological testing comprising the steps of:
   (a) dipping a pad into a hypertonic solution,
   (b) drying the pad,
   (c) inserting the dried pad into an oral cavity to collect immunoglobulins within the oral cavity,
   (d) removing the pad from the oral cavity, and
   (e) eluting the collected immunoglobulins from the pad for analysis by immunological testing.

2. The method of claim 1, wherein the hypertonic solution includes alkali metal salts or alkaline earth metal salts.

3. The method of claim 1, wherein the hypertonic solution includes a blocking agent.

4. The method of claim 3, wherein the blocking agent is albumin or gelatin.

5. The method of claim 1, wherein the hypertonic solution includes a preservative.

6. The method of claim 1, wherein the pad is stored in a container when the pad is removed from the oral cavity.

7. The method of claim 6, wherein the container includes a preservative solution.

8. The method of claim 7, wherein the preservative solution includes chlorhexadine gluconate.

9. The method of claim 1, wherein the hypertonic solution includes a salival stimulating agent.

10. An oral immunoglobulin collecting pad comprising an absorbent material and the salts of a hypertonic solution.

11. The oral immunoglobulin collecting pad of claim 10, further comprising a blocking agent.

12

**12.** The oral immunoglobulin collecting pad of claim 11, wherein the hypertonic solution includes alkali metal salts or alkaline earth metal salts.

**13.** The oral immunoglobulin collecting pad of claim 10, wherein the pad is made of a carbohydrate material.

**14.** An immunological testing kit comprising a pad (3) treated with a hypertonic solution; a pad holder (1); a container (4) for storing the pad (3); a pad removal device (8); and a storage preservative solution (11).

**Patentansprüche**

**1.** Verfahren zur Sammlung von Immunoglobulinen für eine immunologische Prüfung, das folgende Stufen umfaßt:
(a) Eintauchen eines Kissens in eine hypertonische Lösung;
(b) Trocknung des Kissens;
(c) Einführung des getrockneten Kissens in die Mundhöhle zur Sammlung der Immunoglobuline in der Mundhöhle;
(d) Entfernung des Kissens aus der Mundhöhle;
(e) Eluierung der gesammelten Immunoglobuline aus dem Kissen für die Analyse durch immunologische Prüfung;

**2.** Verfahren nach Anspruch 1, wobei die hypertonische Lösung Alkali- oder Erdalkalimetallsalze enthält.

**3.** Verfahren nach Anspruch 1, wobei die hypertonische Lösung ein Blockierungsmittel enthält.

**4.** Verfahren nach Anspruch 3, wobei das Blockierungsmittel Albumin oder Gelatine ist.

**5.** Verfahren nach Anspruch 1, wobei die hypertonische Lösung ein Konservierungsmittel enthält.

**6.** Verfahren nach Anspruch 1, wobei das Kissen in einem Behälter aufbewahrt wird, wenn es aus der Mundhöhle entfernt wird.

**7.** Verfahren nach Anspruch 6, wobei der Behälter eine Konservierungslösung enthält.

**8.** Verfahren nach Anspruch 7, wobei die Konservierungslösung Chlorhexadingluconat enthält.

**9.** Verfahren nach Anspruch 1, wobei die hypertonische Lösung ein die Speichelabsonderung stimulierendes Mittel enthält.

**10.** Orales Kissen zur Sammlung von Immunoglobulin, das einen saugfähigen Stoff und die Salze einer hypertonischen Lösung umfaßt.

**11.** Orales Kissen zur Sammlung von Immunoglobulin nach Anspruch 10, das außerdem noch ein Blockierungsmittel enthält.

**12.** Orales Kissen zur Sammlung von Immunoglobulin nach Anspruch 11, wobei die hypertonische Lösung Alkali- oder Erdalkalimetallsalze enthält.

**13.** Orales Kissen zur Sammlung von Immunoglobulin nach Anspruch 10, wobei das Kissen aus einem Kohlenwasserstoffmaterial hergestellt ist.

**14.** Ausrüstung zur Durchführung einer immunologischen Prüfung, das ein Kissen (3), das mit einer hypertonischen Lösung behandelt wurde; eine Kissenhülse (1); einen Behälter für die Aufbewahrung des Kissens (3); eine Vorrichtung zur Entfernung des Kissens (8); und eine Konservierungslösung für die Aufbewahrung (11) umfaßt.

**Revendications**

**1.** Procédé pour prélever des immunoglobulines, pour essais immunologiques, qui comprend les étapes consistant :

    (a) à immerger un tampon dans une solution hypertonique,

    (b) à sécher le tampon,

    (c) à insérer le tampon séché dans une cavité buccale pour prélever les immunoglobulines se trouvant à l'intérieur de la cavité buccale,

    (d) à extraire le tampon de la cavité buccale, et

    (e) à éluer du tampon les immunoglobulines prélevées, pour analyse par essai immunologique.

**2.** Procédé selon la revendication 1, dans lequel la solution hypertonique comprend des sels de métaux alcalins ou des sels de métaux alcalino-terreux.

**3.** Procédé selon la revendication 1, dans lequel la solution hypertonique contient un agent bloquant.

**4.** Procédé selon la revendication 3, dans lequel l'agent bloquant est l'albumine ou la gélatine.

**5.** Procédé selon la revendication 1, dans lequel la solution hypertonique contient un conservateur.

**6.** Procédé selon la revendication 1, dans lequel le tampon est conservé dans un récipient quand le tampon est extrait de la cavité buccale.

**7.** Procédé selon la revendication 6, dans lequel le récipient contient une solution de conservateur.

**8.** Procédé selon la revendication 7, dans lequel la solution du conservateur contient du gluconate de chlorhexidine.

**9.** Procédé selon la revendication 1, dans lequel la solution hypertonique contient un agent de stimulation de la salive.

**10.** Tampon de prélèvement des immunoglobulines de la cavité buccale, comprenant un matériau absorbant et les sels d'une solution hypertonique.

**11.** Tampon de prélèvement des immunoglobulines de la cavité buccale selon la revendication 10, qui comprend en outre un agent bloquant.

**12.** Tampon de prélèvement des immunoglobulines de la cavité buccale selon la revendication 11, dans lequel la solution hypertonique contient des sels de métaux alcalins ou des sels de métaux alcalino-terreux.

**13.** Tampon de prélèvement des immunoglobulines de la cavité buccale selon la revendication 10, dans lequel le tampon est réalisé en un matériau à base d'hydrates de carbone.

**14.** Trousse d'essai immunologique comprenant un tampon (3) traité par une solution hypertonique ; un porte-tampon (1) ; un récipient (4) pour conserver le tampon (3) ; un dispositif (8) d'extraction du tampon ; et une solution de conservateur (11).

# FIG. Ia

# FIG. Ib

# FIG. 2

# FIG. 3

# FIG. 4